# FASCICULE DE BREVET EUROPEEN

(11) **EP 0 701 456 B1**
(45) Date de publication et mention de la délivrance du brevet: **03.06.1998**
(21) Numéro de dépôt: 93921990.3
(22) Date de dépôt: 04.10.1993
(51) Int. Cl.: A61M 3/02

(54) **DISPOSITIF POUR ALIMENTER UNE CAVITE DU CORPS HUMAIN OU D'UN ANIMAL AVEC UN LIQUIDE SOUS UNE PRESSION DETERMINEE**
VORRICHTUNG ZUR EINBRINGUNG EINER FLÜSSIGKEIT UNTER EINEM BESTIMMTEN DRUCK IN EINE KÖRPERHÖHLE EINES MENSCHEN ODER TIERES
DEVICE FOR SUPPLYING A LIQUID AT A DETERMINED PRESSURE TO A BODY CAVITY OF A PERSON OR AN ANIMAL

(30) Priorité: 01.06.1993 FR 9306627
(43) Date de publication de la demande: 20.03.1996
(73) Titulaire: GUIGNARD, Mireille, F-01630 Saint-Genis Pouilly (FR)
(72) Inventeur: GUIGNARD, Mireille "Le Vézely", F-01630 Saint-Genis/Pouilly (FR); COTTENCEAU, Rémi "Les Hameaux de la Côte", F-74580 Viry (FR); ZUERCHER, Erwin, CH-1219 Le Lignon (CH)
(74) Mandataire: Savoye, Jean-Paul
(86) Numéro de dépôt international: FR9300975
(87) Numéro de publication internationale: WO9427659

(56) Documents cités:
- EP-A- 0 411 170
- FR-A- 2 154 675
- FR-A- 2 592 306
- GB-A- 2 165 312
- US-A- 5 163 909

## Description

La présente invention se rapporte à un dispositif pour alimenter en licuide et mettre une cavité du corps humain ou d'un animal sous une pression determinée en vue d'un examen ou d'une intervention endoscopique, comprenant au moins un sac souole étanche et stérile pour contenir un liquide physiologique, un conduit d'alimentation pour relier ledit sac à ladite cavité, des moyens pour mettre le liquide remplissant ladite cavité sous une pression déterminée, un conduit pour évacuer le quide de cette cavité et des moyens pour régler le débit à travers ce conduit d'évacuation.

Le nombre des examens et des interventions endoscopiques croît constamment. Pour permettre d'obtenir une bonne image de la cavitié du corps à observer ou dans laquelle on doit pratiquer une intervention, on alimente généralement cette cavité en eau physiologique dont le rôle peut être de gonfler la cavité et d'évacuer les matiéres provenant d'une résection des tissus et qui, autrement, rendraient rapidement opaque l'image du champ opératoire. La pression d'alimentation est essentiellement variable en fonction des tissus formant la cavité à inspecter ou dans laquelle on doit intervenir. Le maintien d'une pression constante est de toute première importance, puisqu' aussi bien une augmentation qu'une diminution de la pression dans la cavité peuvent conduire à des répercussions graves pour le patient. En effet, une surpression peut entraîner des infiltrations de liquide dans le corps du patient et des dégats de la cavité elle-même. Une dépression peut avoir d'autres résultats tout aussi graves. C'est ainsi cue, dans une résection d'une tumeur sur une paroi de la vessie, effectuée par une résistance électricue, une baisse subite de pression peut amener intempestivement la paroi de la vessie contre le corps de chauffe et la percer avec toutes les conséquences graves qui en résultent,

Pour mesurer la pression du liquide dans le conduit d'alimentation, on discose un capteur de pression dans le conduit d'alimentation. Un tel capteur pose cependant un problème. Il faut, en effet, ménager une colonne d'air pour en mesurer la pression. A cet effet, on dispose le capteur à l'extrémité d'un conduit latéral placé au-dessus du niveau de liquide et on mesure la pression de l'air emprisonné dans ce conduit latéral. Pour éviter que le liquide physiologique ne puisse venir en contact avec le capteur, un filtre est disposé à une certaine distance de ce capteur. Le danger de ce filtre est qu'au cas où il entre accidentellement en contact avec le liquide, du liquide reste accroché et il se forme ensuite des bulles d'air dans le conduit faussant complètement la mesure. Dès lors, le risque de voir se former une importante surpression n'est pas exclu, avec toutes les conséquences qui en résultent. L'utilisation d'une valve de sécurité n'est pas envisageable dans la mesure où, en cas de fonctionnement, du liquide vient en contact de zones non stériles et qu'il y a alors risque de migration de germes microbiens vers la cavité irriguée. Compte tenu de ces risques, certains praticiens préfèrent utiliser la pression créée par une colonne de liquide de hauteur choisie en fonction de la pression désirée. L'inconvénient vient du fait que, suivant la pression désirée, on ne dispose pas de la hauteur suffisante dans un bloc opératoire.

Jusqu'ici, tous les dispositifs d'irrigation fonctionnant avec une pompe utilisent une pompe péristaltique qui est la seule pompe dont aucune partie mécanique ne vient en contact avec le liquide et qui est donc compatible avec la stérilité du liquide d'alimentation. L'inconvénient de ces pompes est de créer une pression sinusoïdale dans le liquide d'alimentation. Dans certains cas, cette variation sinusoïdale de la pression n'est pas tolérable, comme dans le cas d'une intervention endoscopique dans la trompe utérine où la pression doit être parfaitement constante. Par ailleurs, avec une pompe péristaltique asservie par un capteur de pression, il y a toujours un temps de réponse, de sorte que la pression peut fluctuer et, en réalité, elle fluctue constamment.

On a déjà proposé de mettre un liquide physiologique sous pression en disposant un sachet souple contenant ce liquide dans une enceinte étanche alimentée par un fluide sous pression. Des dispositifs de ce genre sont décrits par le EP-0 411 170, par le US 5,163,909 et par le GB 21 65 312. Le premier de ces documents, le EP-0 411 170, se rapporte à un instrument chirurgical de résection de tumeurs cervicales comprenant un tube d'aspiration servant à aspirer du tissu tumoral et une buse orientée pour envoyer un jet sous haute pression transversalement à l'extrémité du tube d'aspiration, ce jet servant à découper le tissu aspiré, le liquide et le tissu découpé étant aspirés et évacués par le conduit d'aspiration. Les deux autres documents US 5,163,909 et GB 21 65 312 dont destinés à mettre sous pression un liquide destiné à une perfusion en vue de vaincre la pression artérielle. Aucun de ces documents ne se rapporte à l'alimentation et à la mise sous une pression déterminée d'une cavité du corps humain, la pression dans cette cavité étant réglable, constante et indépendante du débit d'alimentation et correspondant à la pression affichée régnant dans l'enceinte étanche. En effet, tous les dispositifs susmentionnés sont destinés à mettre le liquide à injecter sous pression par le fluide introduit dans l'enceinte étanche, mais personne n'a jusqu' ici proposé d'utiliser ce moyen pour régler avec précision et constance la pression que l'on désire faire régner dans une cavité du corps humain indépendamment du débit d'alimentation de cette cavité. En outre, personne n'a de ce fait, proposé de mesurer et d'afficher la pression de l'enceinte de mise sous pression du liquide physiologique pour connaître la valeur de la pression dans la cavité du corps humain irriguée par le liquide physiologique. Or, la connaissance précise de cette pression est importante et varie suivant la nature de la cavité dans laquelle on intervient.

Le but de la présente invention est précisément de remédier au moins en partie aux inconvénients susmentionnés grâce à l'utilisation d'une enceinte de mise sous pression du liquide physiologique alimentée par un fluide sous pression réglable.

A cet effet, la présente invention a pour objet un dispositif pour alimenter en liquide et mettre une cavité du corps humain ou d'un animal sous une pression déterminée selon la revendication 1.

Les avantages du procédé et du dispositif objets de l'invention sont nombreux. Le plus important vient du fait que la pression affichée est réellement la pression dans le conduit d'alimentation et dans la cavité irriguée et que cette pression ne fluctue pas. Au cas, tout à fait improbable, où une surpression se produirait, on peut disposer une soupape de sécurité qui est reliée au fluide de compression et non au liquide d'alimentation, de sorte qu'aucun risque de contamination du liquide d'alimentatin n'existe. Les problèmes susmentionnés, liés à la mesure de la pression, n'existent plus puisque c'est la pression du fluide de compression que l'on mesure et non celle du liquide d'alimentation. Outre la grande fiabilité du dispositif selon l'invention, on peut encore remarquer sa très grande simplicité dans la mesure où il suffit de brancher l'enceinte de mise sous pression du liquide d'alimentation à une source d'air comprimé existant dans chaque bloc opératoire Tout l'asservissement électronique de la pompe péristaltique d'alimentation ainsi que cette pompe ne sont donc plus nécessaires, réduisant ainsi substantiellement le coût de l'appareil.

Le dessin annexé illustre, schématiquement et à titre d'exemple, une forme d'exécution du dispositif pour alimenter une cavité du corps humain ou d'un animal avec un liquide sous une pression déterminée, objet de la présente invention.

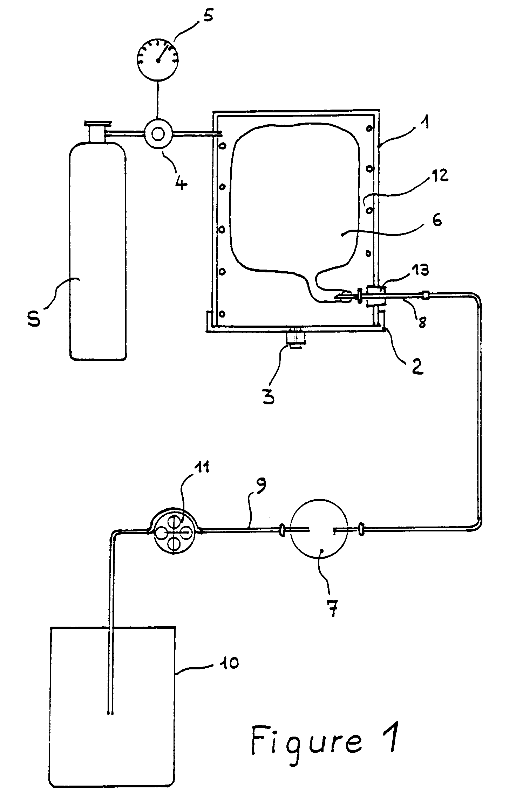

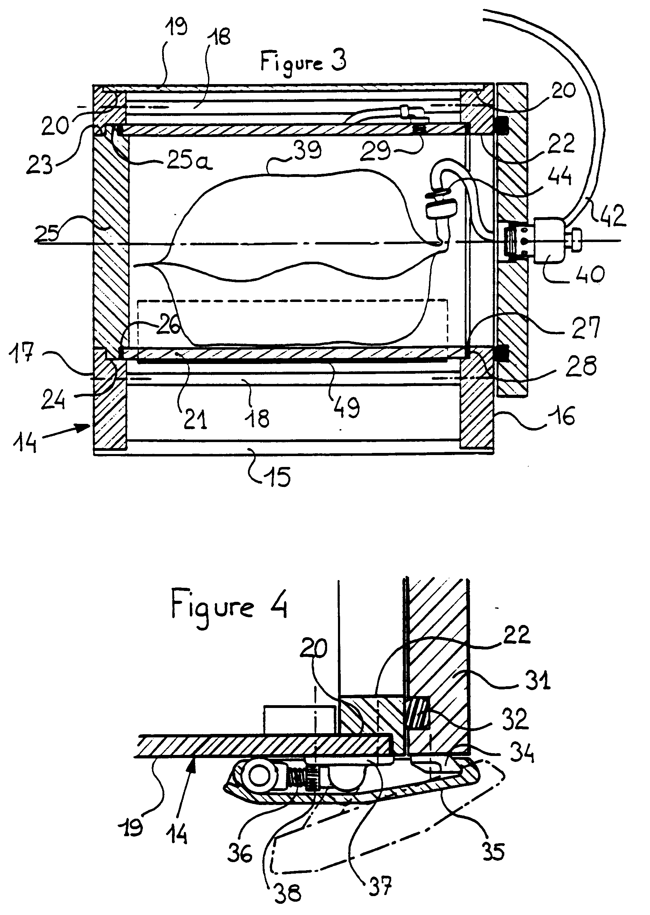

La figure 1 est un sohéma de ce dispositif.

La figure 2 est une vue en perspective d 'une forme d'exécution comprenant deux enceintes de pression en paralléle.

La figure 3 est une vue en coupe selon la ligne III-III de la figure 2.

La figure 4 est une vue en coupe selon la ligne IV-IV de la figure 2.

La figure 5 est une vue en perspective de la face opposée à celle de la figure 2.

Ce dispositif comporte une enceinte étanche 1, à paroi de rigidité suffisante pour résister à la pression destinée à être créée dans cette enceinte. Elle est fermée hermétiquement par un couvercle 2 muni d'une soupape de sécurité 3 tarée en fonction de la pression maximum admissible. Cette enceinte 1 est reliée à une source d'air sous pression S par l'intermédiaire d'une vanne de réglage 4 et d'un régulateur de pression 5. Cette enceinte renferme un sac souple 6 contenant le liquide physiologique destiné à irriguer une cavité 7 du corps humain ou d'un animal. Un conduit 8 traverse la paroi de l'enceinte 1 et sert à relier le sac souple 6 à la cavité 7. Un joint conique 13 sert à assurer l'étanchéité du conduit 8 à travers la paroi de l'enceinte 1. Un conduit d'évacuation 9 relie cette cavité à un bac de récupération 10. Une vanne 11 ou, de préférence, une pompe, sert à régler le débit dans ce conduit 9 en fonction de l ' intervention ou de l 'examen pratiqué par le praticien. Ce débit d'évacuation est déterminé en fonction de la clarté du liquide dans la cavité qui permet au praticien de voir le champ opératoire sur un écran vidéo, de sorte qu'il fait circuler un débit plus ou moins grand en fonction de la netteté de l'image qu'il observe sur cet écran.

Quel que soit le débit, la pression dans l'enceinte 1, et donc dans le sac souple 6, le conduit 8 et la cavité 7, est constante et correspond à celle affichée sur le régulateur de pression 5. Etant donné que la vanne de réglage fonctionne pour maintenir la pression constante, elle s'adapte constamment au débit d'évacuation ainsi qu'aux fuites. Si la pression tend à diminuer, elle effectue instantanément la compensation. Si, par exemple, un corps solide vient à boucher l'évacuation de la cavité 7, ce qui peut se produire en cas de résection endoscopique, il n'y a pas de risque de voir augmenter la pression, même temporairement, étant donné que la pression reste constante tant que le réglage du régulateur de pression 5 n'est pas modifié. La soupape de sécurité 3 n'est là qu'au cas où un organe de réglage viendrait à tomber en panne puisque c'est en fait le seul risque de voir la pression s'élever anormalement. On peut d'ailleurs remarquer qu'il n'était pas possible de munir les systèmes d' irrigation antérieurs d'une valve de sécurité en raison des risques de contamination du liquide d'irrigation en cas de fonctionnement de cette valve invoqués précédemment. Grâce au procédé et au dispositif objet de l'invention, la présence d'une telle valve ne pose pas de problème puisqu'elle intervient sur le fluide compresseur et non sur le circuit d'irrigation lui-même.

Un corps de chauffe 12 peut être disposé dans l'enceinte 1, ce corps de chauffe 12 étant alimenté par un organe de réglage thermostatique (non représenté).

Généralement, le liquide physiologique pour les interventions endoscopiques dans le domaine de l 'arthroscopie est disposé dans deux sacs connectés au même conduit d'alimentation 8. Dans ce cas, il suffit de prévoir une second enceinte, identique à l'enceinte 1, pour y disposer le second sac.

Outre les avantages mentionnés précédemment, on peut encore indiquer que le dispositif objet de la présente invention ne comporte pas d'alimentation électrique autre que le chauffage limité à 40 W, ce oui constitue un avantage pratique non négligeable.

Contrairement aux pompes d' irrigation de l'état de la technique, le temps de réponse est quasi instantané, alors qu' avec les pompes connues, asservies en fonction de la pression mesurée en comprimant une colonne d ' air par le liquide d'alimentation, on additionne deux causes d'inertie. La première est la compression de l'air dans la colonne, la seconde est le temps de répercussion dans le liquide d'alimentation à partir de l'ordre envoyé à la pompe péristaltique. Les essais cliniques effectués à l'aide du dispositif décrit ont parfaitement démontré cet avantage par rapport aux systèmes d'irrigation connus.

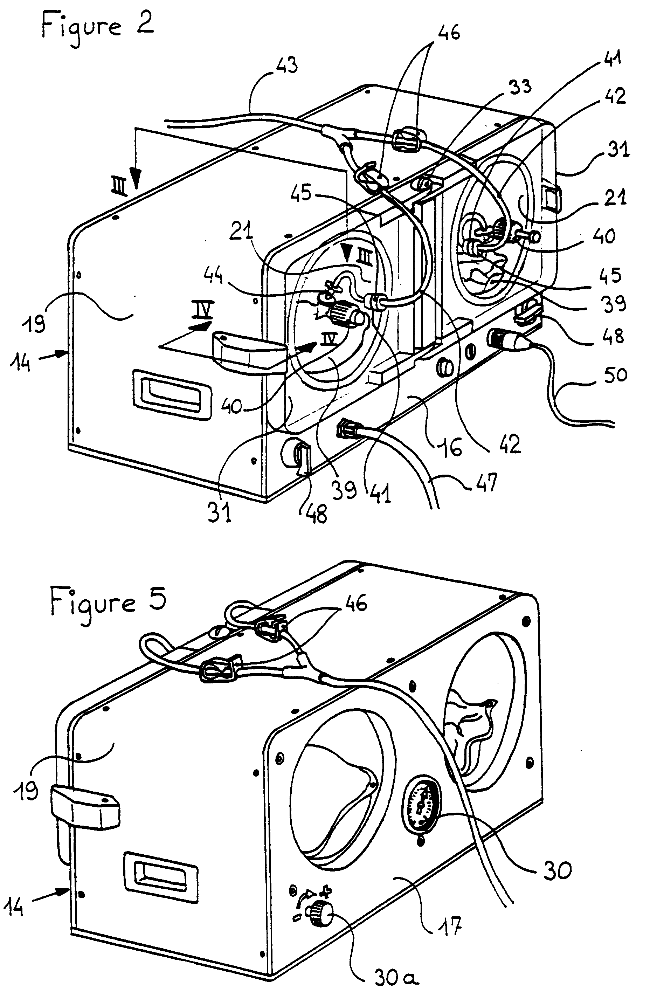

Le dispositif illustré par les figures 2 à 6 comporte un boîtier 14 formé d'un fond 15 de deux parois latérales opposées 16, 17, assemblées par des tirants 18 jouant simultanément le rôle d'entretoises, l'espace interne étant fermé par un couvercle 19 qui vient se loger sur des portées 20 des deux faces opposées 16 et 17.

Deux cylindres 21 sont montés avec leurs axes longitudinaux disposés perpendiculairement aux parois latérales 16 et 17. La figure 3 montre plus en détail le montage des cylindres 21 dans le boîtier 14. Les parois 16 et 17 sont constituées par des plaques percées chacune de deux ouvertures 22, respectivement 23, correspondant sensiblement au diamètre interne des cylindres 21. La paroi arrière 17 présente une portée annulaire interne 24 destinée à recevoir un disque transparent 25, par exemple en plexiglas. Ce disque 25 présente une partie annulaire 25a qui est disposée entre la portée 24 et un joint d'étanchéité 26 comprimé entre une extrémité du cylindre 21 et la partie annulaire 25a. L'autre extrémité du cylindre 21 comprime un joint d'étanchéité 27 contre une portée annulaire interne 28 de la paroi avant 16, l' assemblage des parois 16, 17 et des cylindres étant réalisé par les tirants 18. Chaque cylindre 21 présente une ouverture 29 pour permettre l'introduction d'air sous pression. Cet air provient d'une source d'air comprimé a 600 kPa et entre dans un manomètre-détendeur de précision 30 (fig. 5) réglable à l'aide d'un bouton 30a d'où il ressort à la pression affichée. Dans cet exemple, il s'agit d'un manomètre-détendeur de Festo (Allemagne).

Chaque enceinte ménagée à l'intérieur des cylindres 21 est fermée par une porte transparente 31, dans cet exemple en plexiglas. La face interne de cette porte présente une gorge annulaire dans laquelle un joint d'étanchéité 32 est inséré. La face latérale de cette porte 31 opposée à sa charnière d'articulation 33 présente un ergot d'accrochage 34, tandis que la partie fixe du boîtier 14 porte un organe de fermeture à genouillère comprenant (fig. 4) un élément d'accrochage 35 articulé à l'extrémité d'une tige 36 dont l'autre extrémité est articulée à une embase 37 solidaire du boîtier 14. La longueur de la tige 36 et donc, l'écartement entre les deux axes d'articulation est réglable grâce à un système à écrou de réglage 38. Ce système de fermeture permet de régler le degré de compression du joint d 'étanchéité 32. La position d'ouverture de l'élément d'accrochage 35 a été illustrée en traits mixtes.

Comme illustré par la figure 3, un sachet d'alimentation étanche 39 contenant du liquide physiologique est disposé dans chaque enceinte cylindrique. Chaque porte 31 présente une valve de sécurité 40 réglée à 30 kPa dans le cas où l'appareil est destiné à des interventions orthopédiques par endoscopie, ainsi qu'une ouverture 41 pour permettre le passage étanche d'un conduit d'alimentation 42. Dans ce cas, de façon classique, on a un conduit en Y comprenant deux conduits 42 partant de chacun des sachets 39 d'alimentation aboutissant à un conduit commun 43 conduisant au champ opératoire. Chaque conduit 42 se termine par un percuteur 44 destiné à percer le sachet d'alimentation 39. Entre le percuteur 44 et le conduit commun 43, chaque conduit présente un bouchon d'étanchéité 45 et une pince 46 destinée à contrôler l'ouverture et la fermeture du conduit 42.

La figure 2 illustre encore un conduit d'alimentation en air comprimé 47 et une alimentation électrique basse tension 50 (24 V) pour le chauffage du liquide physiologique, ainsi que des robinets 48 pour commander la mise sous la pression affichée par le manomètre-détendeur 30, des enceintes cylindriques 21.

La figure 3 montre encore une résistance électrique 49 de chauffage du liquide physiologique. Cette résistance 49 et noyée dans une feuille plastique collée contre la face externe de la partie inférieure du cylindre 21. Le chauffage s'effectue par simple contact entre le sachet souple 39, qui épouse la forme de la partie inférieure du cylindre 21, et la partie inférieure de ce cylindre. La résistance est calculée pour accroître la température du liquide physiologique de l'ordre de 18 à 20°C par rapport à la température ambiante afin que la température à la sortie du sachet soit de l'ordre de 42°C, de sorte qu'une fois le liquide arrivé dans la cavité à irriguer, il ait une température de l'ordre de 38° à 40°C.

On peut relever ici différentes particularités intéressantes de cet appareil. Ses deux faces latérales principale opposées sont transparentes, ce qui permet aussi bien au chirurgien qu'à l' instrumentiste de contrôler le niveau du sachet d'alimentation 39. Par ailleurs, une des faces transparentes avec le manomètre-détendeur 30, affichant la pression et permettant au besoin de la modifier grâce au bouton de réglage 30a, peut être tournée vers le chirurgien qui n'a à connaître que la pression et le niveau de remplis-sage du sachet d'alimentation. L'autre face transparente opposée est destinée à être tournée vers l'instrumentiste qui a ainsi accès aux enceintes 21 par les portes transparentes 31, ce qui lui permet d'agir sur l'ouverture et la fermeture des conduits 42 à l'aide des pinces 46 et, au besoin, si deux sachets de liquide physiologique s'avéraient insuffisants, d'en remplacer un.

A cet effet, il lui suffit d'arrêter l'alimentation de l'enceinte 21 correspondante à l'aide du robinet 48, de vider l 'air de l 'enceinte en tirant sur la valve de sécurité correspondante 40 et d'ouvrir la porte 31. Il enlève le percuteur 44 du sachet vide, sort le sachet 39, le remplace par un sachet plein, enfonce le percuteur 44, ferme la porte 31 et ouvre le robinet 48. Toute cette intervention se fait sans jamais gêner le chirurgien puisqu'elle se fait sur la face située, pour celui-ci, vers l'arrière de l'appareil.

Les portes 31 sont réalisées dans une plaque de plexiglas épaisse, ce qui lui donne une rigidité suffisante pour obtenir l'étanchéité avec un seul point de fermeture.

Les tubulures 42 avec bouchon d'étanchéité 45 incorporé permettent d'introduire le percuteur 44 à travers l'ouverture 41 de la porte 31 et de fermer cette ouverture en enfonçant le bouchon 45.

Le montage des faces avant 16 et arrière 17 a l'aide de tirants servant d'entretoise donne un montage extrêmement simple de l'ensemble. L'alimentation basse tension avec une puissance de 40 W permet de réduire l'ampérage au-dessous de 2 A, de sorte que l 'appareil ne nécessite pas d'homologation électrique. Les cylindres, les fonds et les portes des enceintes cylindriques, tous en plexiglas, permettent de réaliser une bonne isolation thermique du liquide physiologique.

Il est également a mentionner le chauffage du liquide physiologique par contact entre la surface inférieure des cylindres 21 et les sachets d'alimentation 39. Ce mode de chauffage sans bain-marie présente un avantage important. Le bain-marie constitue en effet un milieu idéal pour la croissance des germes. Si une partie de la connexion entre le sachet d'alimentation 39 et le percuteur 44 vient accidentellement en contact avec l'eau du bain-marie, il y a un important risque de contamination, risque qui est évité avec le mode de chauffage par la surface du cylindre 21 dont la face externe est chauffée par la résistance 49.

## Revendications

1. Dispositif pour alimenter en liquide et mettre une cavité (7) du corps humain ou d'un animal sous une pression déterminée, en vue d'un examen ou d'une intervention endoscopique, comprenant au moins un sac souple (6,39) étanche et stérile pour contenir un liquide physiologique, un conduit d'alimentation (8,42,43) pour relier ledit sac à ladite cavité, des moyens pour mettre le liquide remplissant ladite cavité (7) sous une pression déterminée, un conduit (9) pour évacuer le liquide de cette cavité (7) et des moyens (11) pour régler le débit à travers ce conduit d'évacuation, caractérisé en ce que lesdits moyens pour mettre le liquide remplissant ladite cavité (7) sous une pression déterminée comprennent au moins une enceinte étanche (1,21,25,31) à paroi rigide vis-à-vis des pressions destinées à y régner, une ouverture pour introduire ledit sac souple, des moyens de fermeture étanche (2,31,32) de cette ouverture, des moyens d'étanchéité (13,45) pour le passage dudit conduit d'alimentation (8,42) hors de cette enceinte, une source (S) de fluide sous pression, un conduit (47) pour relier cette source à ladite enceinte étanche, et des moyens (4,5,30,30a) pour régler la pression dans ladite enceinte en fonction de la pression désirée dans ladite cavité (7).

2. Dispositif selon la revendication 1, caractérisé par le fait que l'enceinte est munie d'une valve de sécurité (3,40) réglable.

3. Dispositif selon la revendication 1, caractérisé par le fait que l'enceinte est munie de moyens de chauffage (12,49) thermostabilisés.

4. Dispositif selon la revendication 1, caractérisé par le fait qu'il comporte un boîtier parallélipipédique (14) dont deux faces latérales opposées (16,17) sont transparentes et entre lesquel les sont disposées deux enceintes cylindriques (21) dont les extrémités sont adjacentes auxdites faces latérales transparentes (16,17), des joints d'étanchéité (26,28) étant disposés entre ces faces et les extrémités desdites enceintes, l'une de ces faces latérales (16) comprenant deux portes (31), une pour chaque enceinte, ces portes présentant chacune une ouverture (41) pour le passage étanche d'un conduit d'alimentation, ces conduits aboutissant à un conduit d'irrigation commun (43).

5. Dispositif selon la revendication 4, caractérisé par le fait que lesdites enceintes cylindriques (21) sont en un matériau isolant thermique transparent.

6. Dispositif selon la revendication 4, caractérisé par le fait qu'une résistance électrique (49) est solidaire d'un moins une portion de la paroi inférieure de chacune desdites enceintes cylindriques (21).

7. Dispositif selon la revendication 1, caractérisé par le fait qu'un bouchon d'étanchéité face (45) est monté le long dudit conduit (42) et est destiné à assurer le passage étanche dudit conduit à travers ladite ouverture (41) de l'enceinte.

8. Dispositif selon les revendications 4 et 7, caractérisé par le fait que chacun desdits conduits (42) aboutissant à un conduit d'irrigation commun (43) se termine par un percuteur (44) destiné à traverser la paroi dudit sac et par le fait qu'un bouchon d'étanchéité percé (41) est monté le long de chacun de ces conduits entre le percuteur (44) et ledit conduit d'irrigation commun (43).

9. Dispositif selon la revendication 4, caractérisé par le fait que chaque porte (31) est fermée par un système de fermeture à genouillère (34-38).

10. Dispositif selon la revendication 4, caractérisé par le fait qu'un manomètre de réglage (30,30a) de la pression dans lesdites enceintes (21) est disposé sur la face latérale (17) du boîtier (14) opposée à la face (16) comportant lesdites portes (31).

11. Dispositif selon la revendication 4, caractérisé par le fait que lesdites faces latérales (16,17) opposées sont fixées l'une à l'autre par des tirants (18) servant simultanément d'entretoise d'écartement.

## Claims

1. A device to supply a liquid to a cavity(7) of a person or an animal and pressurize it to a determined pressure, for the purpose of an endoscopic examination or operation, made up of at least one watertight arid sterile flexible bag (6, 39) to contain a biological liquid, a supply tube (8, 42, 43) to connect the said hag to the said cavity by a watertight and sterile tube, the means to pressurize the liquid fitting the said cavity (7) at a determined pressure, a tube (9) to evacuate the liquid from this cavity (7) and the means (11) to adjust the flow rate through this outlet tube, characterized in that the said moans to pressurise the liquid filling the said cavity (7) at a determined pressure include at least one watertight enclosure (1,21, 25, 31), with rigid walls with respect to the pressures it will have to contain, an opening to insert the said flexible bag, the means to achieve a watertight seal (2, 31, 32) of this opening, the means to achieve a watertight seal for the exit of the said supply tube (8, 42) from this enclosure, a source (S) of this pressurized fluid, a tube (47) to connect this source to the said watertight enclosure and the means (4, 5, 30, 30a) to adjust the pressure in the said enclosure, depending on the pressure desired in the said cavity (7).

2. A device pursuant to claim 1, characterized by the fact that the enclosure is equipped with an adjustable security valve (3, 40).

3. A device pursuant to claim 1, characterized by the fact that the enclosure is equipped with a thermoresistant heating mechanism (12, 49).

4. A device pursuant to claim 1, characterized by the fact that it has a parallepipedic housing (14), of which two opposed lateral walls (16, 17) are transparent and between which are installed two cylindrical enclosures (21), the ends of which are adjacent to the said transparent lateral walls (16, 17), sealing gaskets (26, 28) being inserted between these walls and the ends of the said enclosures, one of these lateral walls (16) having two doors (31), one for each enclosure, these doors each presenting one opening (41) for the watertight passage of a supply tube, these tubes ending at a common irrigation tube (43).

5. A device pursuant to claim 4, characterized by the fact that the said cylindrical enclosures (21) are made of thermal insulating material.

6. A device pursuant to claim 4, characterized by the fact that an electrical resistance (49) is interdependent with at least a portion of the lower wall of each of the said cylindrical enclosures (21).

7. A device pursuant to claim 1, characterized by the fact that a sealing plug (45) is mounted on the said tube (42) and is meant to ensure the watertight passage of the said tube through the enclosure's said opening (41).

8. A device pursuant to claims 4 and 7, characterized by the fact that each of the said tubes (42) ending at a common irrigation (43) tube has a strike pin (44) at one end, meant to pierce the wall of the said bag and by the fact that a holed sealing plug (41) is mounted on each of these tubes between the strike pin and the said common irrigation tube (43).

9. A device pursuant to claim 4, characterized by the fact that each door (31) is closed by a toggle type fastening system (34-38).

10. A device pursuant to claim 4, characterized by the fact that a pressure adjustment manometer (30, 30a) in the said enclosures (21) is installed on the lateral wall (17) of the housing (14), opposite the wall (16) with the said doors (31).

11. A device pursuant to claim 4, characterized by the fact that the said opposed lateral walls (16, 17) are fixed to each other by tie rods (18), also acting as a spacer frame.

## Patentansprüche

1. Vorrchtung, um eine Körperhöhle eines Menschen oder Tieres zum Zweck einer endoskopischen Untersuchung oder Operation mit Flüssigkeit zu beschicken und unter einen bestimmten Druck zu setzen, mit zumindest einem weichen, dichten und sterilen Beutel (6, 39) für physiologische Lösung, einem Beschickungsrohr (8, 42, 43), um den benannten Beutel mit der benannten Höhle zu verbinden, Organen, um die die benannte Höhle (7) ausfüllende Flüssigkeit unter einen bestimmten Druck zu setzen, einer Rohrleitung (9), um die Flüssigkeit aus dieser Höhle (7) zu entleeren, und Organen (11), um die Durchflussmenge in dieser Entleerungsleitung einzuregeln, dadurch gekennzeichnet, dass die benannten Mittel, die dazu dienen, die die benannte Höhle (7) ausfüllende Flüssigkeit unter einen bestimmten Druck zu setzen, zumindest eine dichte Kammer (1, 21, 25, 31) mit gegenüber den darin vorgesehenen Drücken starren Wänden, eine Öffnung zur Einführung des benannten weichen Beutels, Organe (2, 31, 32) zum dichten Verschluss dieser Öffnung, Dichtungsorgane (13, 45) für den Austritt des benannten Beschickungsrohres (8, 42) aus dieser kammer, eine Quelle (S) für Fluid unter Druck, eine Leitung (47) zum Anschluss dieser Quelle an die benannte dachte Kammer sowie Organe (4, 5, 30, 30*a*) umfassen, um den Druck in der benannten kammer in Abhängigkeit von dem in der benannten Höhle (7) gewünschten Druck einzuregeln.

2. Vorrichtung gemäss Anspruch 1, dadurch gekennzeichnet, dass die kammer mit einem einstellbaren Sicherheitsventil (3, 40) versehen ist.

3. Vorrichtung gemäss Anspruch 1, dadurch gekennzeichnet, dass die kammer mit thermisch stabilisierten Heizorganen (12, 49) versehen ist.

4. Vorrichtung gemäss Anspruch 1, dadurch gekennzeichnet, dass sie ein quaderförmiges Gehäuse (14) umfasst, dessen zwei gegenüberliegende Seitenwände (16, 17) durchsichtig sind und wo zwischen diesen Wänden zwei zylindrische Kammern (21) angeordnet sind, deren Enden an die benannten durchsichtigen Seitenwände (16, 17) angrenzen, wobei Dichtungen (26, 28) zwischen diesen Wänden und den Enden der benannten Kammern angeordnet sind, eine (16) dieser Seitenwände zwei Türen (31) hat, und zwar für jede Kammer eine, diese Türen je eine Öffnung (41) für die dichte Durchführung eines Beschickungsrohres aufweisen und diese Rohre in ein gemeinsames Irrigationsrohr (43) münden.

5. Vorrichtung gemäss Anspruch 4, dadurch gekennzeichnet, dass die benannten zylindrischen Kammern (21) aus einem durchsichtigen, thermisch isolierenden Werkstoff bestehen.

6. Vorrichtung gemäss Anspruch 4, dadurch gekennzeichnet, dass ein elektrischer Widerstand (49) fest mit zumindest einem Abschnitt der Unterseite jeder der benannten zylindrischen Kammern (21) verbunden ist.

7. Vorrichtung gemäss Anspruch 1, dadurch gekennzeichnet, dass ein durchbohrter Dichtungsstopfen (45) über das benannte Rohr (42) gezogen worden und dazu bestimmt ist, die dichte Durchführung des benannten Rohres durch die benannte Öffnung (41) der Kammer zu gewährleisten.

8. Vorrichtung gemäss Ansprüchen 4 und 7, dadurch gekennzeichnet, dass jedes der benannten Rohre (42), die in ein gemeinsames Irrigationsrohr (43) münden, am Ende einen Stichel (44) tragen, der dafür bestimmt ist, die Wandung des benannten Beutels zu durchstossen, und dadurch, dass ein durchbohrter Dichtungsstopfen (41) über jedes dieser Rohre zwischen dem Stichel (44) und dem benannten gemeinsamen Irrigationsrohr (43) gezogen worden ist.

9. Vorrichtung gemäss Anspruch 4, dadurch gekennzeichnet, dass jede Tür (31) durch ein Verschlussystem mit Kniegelenk (34 - 38) verschlossen wird.

10. Vorrichtung gemäss Anspruch 4, dadurch gekennzeichnet, dass ein Manometer (30, 30*a*) für die Regelung des Druckes in den benannten Kammern (21) an der Seitenwand (17) des Gehäuses gegenüber der Seite (16) mit den benannten Türen (31) angeordnet ist.

11. Vorrichtung gemäss Anspruch 4, dadurch gekennzeichnet, dass die benannten gegenüberliegenden Seitenwände (16, 17) aneinander durch Ankerbolzen (18) befestigt sind, die gleichzeitig als Distanzstreben dienen.
